Europäisches Patentamt

(19) European Patent Office

Office européen des brevets

(11) Numéro de publication : **0 246 978 B1**

## (12) FASCICULE DE BREVET EUROPEEN

(45) Date de publication du fascicule du brevet :
**09.01.91 Bulletin 91/02**

(51) Int. Cl.$^5$ : **G01N 1/28, G01N 33/04**

(21) Numéro de dépôt : **87401149.7**

(22) Date de dépôt : **21.05.87**

---

(54) **Réactif pour rendre transparents des milieux biologiques et ses applications analytiques.**

---

(30) Priorité : **21.05.86 FR 8607236**

(43) Date de publication de la demande :
**25.11.87 Bulletin 87/48**

(45) Mention de la délivrance du brevet :
**09.01.91 Bulletin 91/02**

(84) Etats contractants désignés :
**AT BE CH DE ES GB GR IT LI LU NL SE**

(56) Documents cités :
**EP-A- 0 004 857**
**FR-A- 2 187 271**
**FR-A- 2 393 291**
**GB-A- 2 084 726**

(73) Titulaire : **UNIVERSITE DE NANCY I**
**24, rue Lionnois, B.P. 3137**
**F-54013 Nancy Cédex (FR)**

(72) Inventeur : **Linden, Guy**
**21, Place St Malo**
**F-54180 Heillecourt (FR)**
Inventeur : **Humbert, Gérard Marie**
**11, rue Paul Valéry**
**F-54140 Jarville La Malgrange (FR)**
Inventeur : **Kouomegne, Rosalie s/c Ms R**
**Yongue BP 812**
**Fac des Sciences Dept des Sciences de la**
**Terre**
**Université de Yaounde Yaounde (CM)**
Inventeur : **Guingamp, Marie France**
**17, rue du Maréchal Ney**
**F-54180 Heillecourt (FR)**

(74) Mandataire : **Peaucelle, Chantal et al**
**S.C. Ernest Gutmann - Yves Plasseraud 67,**
**boulevard Haussmann**
**F-75008 Paris (FR)**

## Description

L'invention a pour objet un réactif pour la transparisation de milieux biologiques et ses applications analytiques.

On sait que de nombreux milieux biologiques présentent une opalescence naturelle rendant impossible toute mesure spectrophotométrique directe.

Un traitement préalable doit être appliqué pour transpariser le milieu, c'est-à-dire pour le rendre transparent, en lui conférant une faible absorbance, inférieure à 0,1 unité de densité optique entre 400 et 800 nm environ.

Dans le cas par exemple du lait, la turbidité observée est due essentiellement à la présence de phosphocaséinates de calcium et aux globules gras.

Des réactifs, constitués par des mélanges de solvants, ont été proposés pour la dissolution des constituants colloïdaux.

Ainsi, Bosset et al ont étudié la dissolution intégrale du lait au moyen de solvants mixtes tels que les mélanges soude/eau/n-butylamine ou soude/eau/tétrahydrofurane (voir références bibliographiques (1) et (2) en fin de description).

Ces solvants permettent de doser les protéines totales du lait par la méthode dite de Biuret, mais présentent l'inconvénient de rendre nécessaire une forte dilution du lait.

Un autre réactif de transparisation du lait a été décrit par Linden et al (3). Ce réactif, appelé ci-après réactif de Linden, est constitué par un mélange de n-butylamine/cyclohexanone/octylphénol de polyoxyéthylène (tel que celui commercialisé sous la marque Triton X). Il présente l'avantage d'entraîner une transparisation rapide en ne nécessitant qu'une faible dilution.

Toutefois, la viscosité, même dans le mélange final transparisé, est élevée, de l'ordre de 1,1 Pl à 20°C, ce qui le rend difficile à manipuler. En outre, son agressivité s'avère importante vis-à-vis de polymères plastiques. Les instruments de mesure à base de tels polymères ne peuvent donc pas être utilisés. L'agressivité de ce solvant s'exerce également à l'égard des bactéries présentes dans le milieu biologique. Il faut alors effectuer les mesures rapidement, avant la lyse des cellules. De plus, de par sa composition, ce réactif ne permet pas de déterminer certains paramètres. Ainsi il n'est pas approprié à la détermination du taux de groupements aminés dans un échantillon étant donné qu'il renferme lui-même une amine qui peut interférer lors d'une mesure avec ceux de l'échantillon.

Les développements des travaux des inventeurs dans ce domaine les ont amené à élaborer un nouveau réactif applicable à la transparisation du lait et de tout autre milieu biologique sous forme d'émulsion ou de suspension. Ce réactif dépourvu de n-butylamine, constituant principal du réactif de Linden décrit ci-dessus, comporte une combinaison de constituants permettant d'obtenir des effets nouveaux avantageux. En particulier, les inventeurs ont constaté que l'utilisation en combinaison de certains constituants permet de disposer d'un réactif de transparisation de plus grande stabilité, d'agressivité moindre, de viscosité réduite et de supprimer les incompatibilités dans la détermination de certaines fonctions dans des milieux biologiques.

L'invention a donc pour but de fournir un nouveau réactif de transparisation permettant d'éliminer instantanément la turbidité d'un milieu biologique donné, plus aisé à manipuler grâce à une viscosité réduite et de très grande stabilité.

Elle vise également l'utilisation de ce réactif pour réaliser des analyses physiques, chimiques et biologiques de milieux biologiques opalescents, plus spécialement de produits alimentaires ou de suspensions obtenues à partir de produits alimentaires ou encore de produits cosmétiques.

Le réactif de transparisation de l'invention, à base d'un mélange de solvants, est caractérisé en ce qu'il renferme en combinaison :
- une cétone aliphatique constituant un solvant lipophile, soluble en milieu aqueux, stable et permettant une mesure dans le proche UV,
- un agent détergent tensio-actif jouant le rôle de liant, à base d'un dérivé de polyoxyalcoylène non ionique ou d'un sel alcalin amphotère,
- une base et/ou un agent tensio-actif ayant un effet dénaturant vis-à-vis des structures conformationnelles des constituants du milieu biologique à analyser.

D'une manière avantageuse, l'utilisation en combinaison de ces éléments conduit à un réactif permettant d'obtenir instantanément la transparisation d'un milieu donné, de stabilité améliorée par rapport aux réactifs connus.

De plus, on observera que ce réactif est dépourvu de n-butylamine ce qui permet d'éviter les inconvénients d'odeur de ce produit et toute interférence avec les amines d'un milieu dont on souhaite déterminer le taux en amines. Contrairement aux réactifs connus, il n'entraîne pas d'hydrolyse du réactif d'Ellman, le dithio-bis-2-nitrobenzoyle ou DTNB et permet ainsi de déterminer le taux de SH ou de S-S par exemple du lait.

De préférence, la cétone aliphatique est choisie parmi les cétones présentant une constante diélectrique $\Sigma$ de l'ordre de 18,5.

Il s'agit notamment de cétones à chaîne linéaire, renfermant au plus 4 atomes de carbone. Une cétone préférée est constituée par la butanone-2.

Cette cétone présente l'avantage d'une densité relativement faible et d'un paramètre de solubilité dans l'eau élevé.

Un autre cétone appropriée est constituée par la pentane-dione.

L'agent détergent tensio-actif est un dérivé de polyoxyalcoylène non ionique du type éther, alcool ou ester ou un sel alcalin amphotère.

Cet agent sert de liaison entre les phases aqueuses, contenant les protéines, et les phases lipidiques.

Un dérivé particulièrement préféré est le polyoxyéthylène p, t-octylphénol tel que celui commercialisé sous la marque Triton X.

Ce produit présente l'avantage d'être le moins dénaturant, le plus aisément disponible et d'un faible coût.

D'autres dérivés de polyoxyalcoylène sont cependant appropriés, parmi lesquels on citera :
- le polyoxyéthylène nonylphénol tel que celui commercialisé sous la marque Triton N,
- le polyoxyéthylène alcool tel que celui commercialisé sous la marque Brij,
- le polyoxyéthylène ester de sorbitol tel que celui commercialisé sous la marque Tween,
- le polyoxyéthylène ester d'acide gras (monolaurate) tel que celui commercialisé sous la marque Span.

Parmi les sels alcalins amphotères, on citera le désoxycholate de sodium et le diiodosalicylate de lithium.

Comme indiqué ci-dessus, le réactif de l'invention renferme également, en combinaison avec le solvant lipophile et l'agent détergent tensio-actif définis dans ce qui précède, une base et/ou un agent tensio-actif à effet dénaturant.

De préférence, la base est constituée par de la soude.

On constate en effet que les ions sodium exercent un effet favorable sur la désagrégation des micelles des protéines du milieu biologique à analyser et les rendent solubles.

L'agent tensio-actif est un sel de sodium, de préférence en particulier le dodécyl sulfate de sodium.

Un tel produit permet de neutraliser les charges électriques des protéines ce qui supprime les interactions entre les protéines.

Selon un mode préféré de réalisation de l'invention, le réactif de transparisation comprend de la butanone-2, du Triton X, de la soude et/ou du dodécylsulfate de sodium.

La combinaison de ces éléments conduit à un réactif stable à température ambiante, qui se conserve bien à l'état congelé.

Au contact de cette combinaison, on constate également une grande stabilité du milieu biologique à étudier ce qui laisse le temps de faire les mesures nécessaires en série.

Un autre avantage du réactif réside dans sa viscosité, de l'ordre de 0,97 PI à 20°C, qui est donc réduite par rapport à celle du réactif à base de n-butylamine de l'art antérieur décrit ci-dessus.

Les proportions relatives de ces constituants sont ajustées en fonction du taux de protéines et de lipides du milieu biologique à analyser. Ainsi, avec un milieu pauvre en lipides le taux de solvant lipophile pourra être considérablement abaissé.

D'une manière générale, les quantités de solvant lipophile et d'agent tensio-actif détergent sont ajustées l'une par rapport à l'autre. De même la quantité de base est ajustée par rapport à celle de l'agent tensio-actif dénaturant.

L'un de ces groupes, à savoir solvant lipophile et agent détergent tensio-actif d'une part, base et/ou agent dénaturant d'autre part est présent à raison d'au moins 15%, et d'au plus 85%.

On obtient une réaction de transparisation appropriée en mélangeant tout d'abord le solvant lipophile à raison de 15 à 85% en volume, et l'agent détergent tensio-actif à raison de 85 à 15% en volume. La base est utilisée à raison de 0 à 0,5N et l'agent dénaturant à raison de 15 à 0% en volume.

Grâce à son pouvoir dissolvant élevé, le réactif de l'invention s'avère particulièrement approprié à la transparisation de milieux biologiques aux fins d'analyses. Il permet en effet une dissolution intégrale des constituants protéiques et lipidiques et ne nécessite qu'une faible dilution. Il est ainsi possible d'effectuer rapidement et directement des dosages spectrométriques et ce jusque dans le proche UV étant donné sa faible absorbance entre 330 et 800 nm.

Parmi les applications analytiques, on peut citer:
- le dosage de groupes spécifiques, tels que des fonctions amines afin d'étudier notamment l'affinage de fromages à pâte molle, des groupements sulfhydriles et des ponts disulfures, ce qui permet d'apprécier le degré de chauffage d'un échantillon, d'éléments minéraux par exemple Ca, Fe, Mg, Cl ou $NO_3$,.
- la détermination des activités enzymatiques par exemple, dans le lait et les produits laitiers, de la phosphatase alcaline, de la peroxydase pour contrôler un traitement thermique, des protéinases et de la β-D-galactosidase pour contrôler par exemple la qualité de la matière première,
- le dénombrement des micro-organismes présents dans un milieu avec identification, l'étude des courbes de croissance de germes. Ces études permettent notamment d'estimer la vitesse de croissance de bactéries lactiques dans un levain de laiterie, d'identifier, par analyse d'image, certaines souches, après transparisation de laits, levains, yaourts ou autres produits laitiers, de mettre en évidence l'agressivité du réactif de transparisation envers les bactéries (l'observation au microscope électronique de la lyse de cellules apparaît beaucoup moins rapide qu'avec les réactifs connus).

Ces mesures peuvent être effectuées sur tout produit renfermant des protéines et des lipides, plus spécialement des produits alimentaires tels que les produits laitiers, les laits entiers, écrémés, demi-écrémés, en poudre, le lactosérum, les fromages, les levains, les produits carnés, les produits en émulsion d type eau dans l'huile, ou encore des produits cosmétiques notamment les crèmes, aux fins de vérification de l'absence de produits toxiques.

D'autres caractéristiques et avantages de l'invention apparaitront dans les exemples qui suivent, et en se référant aux figures 1 à 5.

- la figure 1 représentant les photos d'une bactérie du lait avant transparisation, 1 heure après transparisation respectivement avec le réactif antérieur de Linden et avec le réactif de l'invention,
- la figure 2, des courbes concernant les propriétés spectrales de différents laits industriels transparisés,
- la figure 3, la courbe obtenue en dosant le taux de groupes $NH_2$ dans des fromages, et
- les figures 4 et 5, respectivement les courbes spectrométriques et néphélémétrique de vitesse de croissance d'une bactérie lactique.

Exemple 1 :

Préparation d'un réactif de transparisation et application à l'analyse du lait.

On élabore un réactif formé du mélange quaternaire suivant :
- soude 0,1 N,
- butanone -2,
- Triton X-100,
- SDS à 1%.

Dans un tube à essai, on ajoute 2 ml de ce mélange à 1 ml d'échantillon de lait écrémé ou demi-écrémé, ou 0,5 ml d'échantillon de lait entier ou 1 ml de suspension dans l'eau chaude de produits laitiers (yaourts à 10%, fromages à pâtes fraîches à 10%, fromages à pâtes molles ou à pâtes pressées à 5%, crème à 5% en émulsion). On incube 2 à 5 min dans un bain-marie à 37°C ± 1.

L'absorption résiduelle, dans le cas d'un lait écrémé varie de 0,010 à 0,098 entre 800 et 330 nm ce qui permet d'effectuer des réactions colorimétriques qui peuvent être lues au spectromètre d'absorption moléculaire.

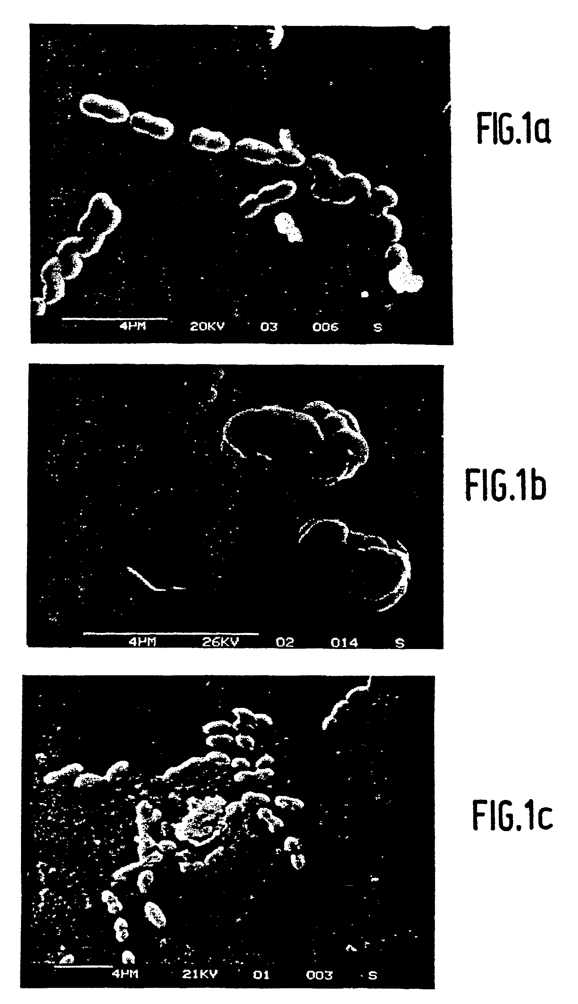

Sur la figure 1, on a représenté les photos
(a) de spectrococcus thermophilus CNRZ 302 présente dans le lait,
(b) de ces bactéries 1 heure après transparisation par le réactif de Linden de l'art antérieur,
(c) 1 heure après transparisation avec le réactif de l'invention.

Les résultats obtenus montrent que les bactéries ne sont pratiquement pas lysées lorsqu'on utilise le réactif de l'invention (voir photo 1c) alors que cette lyse apparaît nettement en examinant la photo 1b obtenue après transparisation avec le réactif connu.

Ces résultats mettent donc en évidence la faible agressivité du réactif de l'invention.

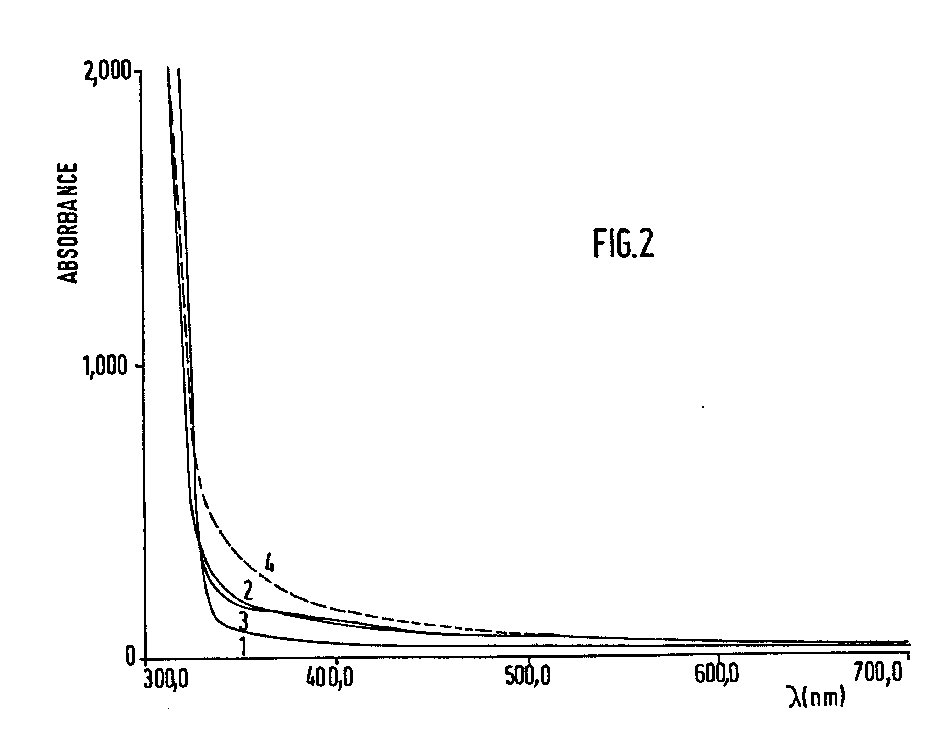

Sur la figure 2, on a rapporté les courbes illustrant les propriétés spectrales de différents laits industriels transparisés. La courbe 1 correspond à celle du réactif pur et les courbes 2, 3 et 4 à celles d'un lait frais écrémé, d'un lait UHT écrémé et d'un lait surchauffé écrémé. L'examen de ces courbes montre la faible absorbance du réactif de l'invention.

Exemple 2 :

Application du réactif de l'exemple 1 au dosage d'enzymes dans le lait.

Application au dosage de l'activité phosphatasique :

1° Principe

La phosphatase alcaline hydrolyse son substrat, le p-nitrophénylphosphate disodique. La libération du p-nitrophénol est suivie par spectrométrie après dissolution totale de tous les composants du milieu réactionnel par le réactif de l'invention.

2° Réactifs

On utilise les réactifs suivants :
- solution aqueuse tampon de diéthanolamine $24,3 \cdot 10^{-3}$ mol/l, pH 10,6,
- substrat : p-nitrophénylphosphate $5 \cdot 10^{-3}$ mol/l final dans la solution tampon décrite ci-dessus,
- réactif de l'exemple 1.

3° Mode opératoire

On introduit dans un tube à essai 0,5 ml de lait dilué au 1/100ème et 1 ml d'une solution substrat. Après agitation, on laisse incuber à 37°C pendant 15 min, puis on ajoute 2 ml de réactif. On agite, et on lit dans un intervalle de temps de 5 min l'extinction de la solution à 420 nm, à 37°C.

4°Expression des résultats

On dose un témoin sans incubation :
L'activité phosphatasique est exprimée en $\mu$ mole de p-nitrophénol libérée par ml de lait en fonction du temps.

La valeur moyenne trouvée sur 22 laits de grand mélange est de 55 $\mu$ moles de 4 nitrophénol libéré/ml lait/15 min.

Application au dosage de l'activité peroxydasique :

1° Principe

Cette méthode repose sur la décomposition de l'eau oxygénée par la peroxydase et fixation de l'oxygène atomique sur un accepteur.

La mesure du colorant ainsi produit se fait par spectrométrie après dissolution totale des divers composants du milieu réactionnel par le réactif de l'invention.

## 2° Réactifs

On utilise les réactifs suivants :
- solution aqueuse de p-phénylène diamine,2HCl à 2% filtrée (P.E.D.),
- eau oxygénée à 10% V/V,
- solution aqueuse de NaOH 3,1 mol/l,
- réactif de l'exemple 1 à pH apparent 13,7.

## 3° Mode opératoire

On amène le pH du P.E.D. à 7 avec la solution aqueuse de NaOH ci-dessus.

On introduit dans un tube à essai 0,5 ml de lait dilué au 1/10ème, puis on ajoute 0,5 ml de la solution de P.E.D. pH 7, puis 0,2 ml de la solution $H_2O_2$.

Après agitation, on ajoute 0,2 ml de la solution NaOH et 2 ml du réactif de l'invention.

On agite le mélange et on lit l'extinction de la solution à 470 nm, à 37°C.

On place un témoin avec du lait bouilli en suivant le même mode opératoire dans la cuve de référence du spectromètre.

## 4° Résultats

La mesure de l'activité peroxydasique est exprimée en unité activité arbitraire par ml de lait en fonction du temps

Une unité arbitraire par ml de lait est égale à une augmentation de l'absorbance de 1/1000ème d'unité selon les conditions opératoires précédentes.

La valeur moyenne observée sur 22 laits de grand mélange = 410 unités.

## Application au dosage de l'activité protéinasique par l'utilisation des substrats synthétiques spécifiques.

### 1° Principe

Les activités protéolytiques sont mises en évidence par l'emploi de différents substrats de synthèse appartenant à la série des 4-nitroanilides, qui sont hydrolysés au cours de l'incubation. La mesure se fait par spectrométrie après dissolution totale du milieu réactionnel par le réactif de l'invention.

### 2° Réactifs

On utilise les réactifs suivants :
- solution tampon pH 8 :
  - triethanolamine 0,094 mol/l,
  - azide de sodium 0,02%.
- substrats : L-alanine-4-nitroanilide ou N-acétyl-L-alanine-4-nitroanilide par exemple.

Le substrat est dissous dans le tampon à la concentration de $5 \cdot 10^{-3}$ mol/l final.
- le réactif de l'exemple 1.

### 3° Mode opératoire

On mélange dans un tube à essai 0,5 ml de lait et 1,5 ml du tampon-substrat.

On agite et on laisse incuber de 0 à 24 heures à 37°C.

On ajoute 2 ml de réactif de l'invention, on soumet à agitation puis on laisse 15 min à 37°C.

On procède à une lecture de l'extinction de la solution à 410 nm, à 37°C.

### 4° Expression des résultats

Un témoin est dosé selon le même mode opératoire mais sans incubation.

Les résultats sont exprimés en μ moles de 4-nitroaniline libérée par ml de lait en fonction du temps.

La valeur moyenne trouvée sur 22 laits de grand mélange est de
- 1 000 μmole/ml lait/24 h pour le substrat L-alanine-4-nitroanilide,
- 800 μmole/ml lait/24 h pour le substrat N-acétyl-L-alanine-4-nitroanilide.

## Exemple 3 :

Application du réactif de l'exemple 1 au dosage des groupements sulfhydriles dans les laits.

A 1 ml d'échantillon (lait dilué au 1/2 dans un tampon pH 8 Gly 92 mM-Tris 1mM), on ajoute 1 ml d'urée 8 M (dans le tampon pH 8), puis 50 μl de réactif DTNB (4 mg de 5.5'-dithio-bis-2-nitrobenzoate par ml).

On incube 10 min à température ambiante, puis on ajoute sous agitation 2 ml du réactif de transparisation. On laisse 2 min à 37°C, puis dans un intervalle de 20 mn, on effectue une lecture à 412 nm.

On obtient les résultats suivants :
lait frais écrémé 1,90 μg SH/g de lait,
lait instantané écrémé 1,47 μg SH/g de lait,
lait UHT écrémé 0,55 μg SH/g de lait.

## Exemple 4 :

Application du réactif de l'exemple 1 au dosage de groupements $NH_2$ dans les laits et fromages.

A 1 ml d'échantillon (lait dilué au 1/10e, suspension de fromage à 1% dans l'eau chaude), on ajoute 1 ml de réactif TNBS (acide trinitro-benzène sulfonique à 0,1% dans $NaHCO_3$ 4% pH 8).

On incube 2 h à 37°C, puis on ajoute 1 ml de tampon $NaHCO_3$ 4% pH 8,5, on ajoute 0,5 ml d'HCl 0,1 N, et 1,5 ml de réactif de transparisation.

On laisse le mélange 2 min à 37°C, puis dans un intervalle de 30 min, on effectue une lecture à 420 nm.

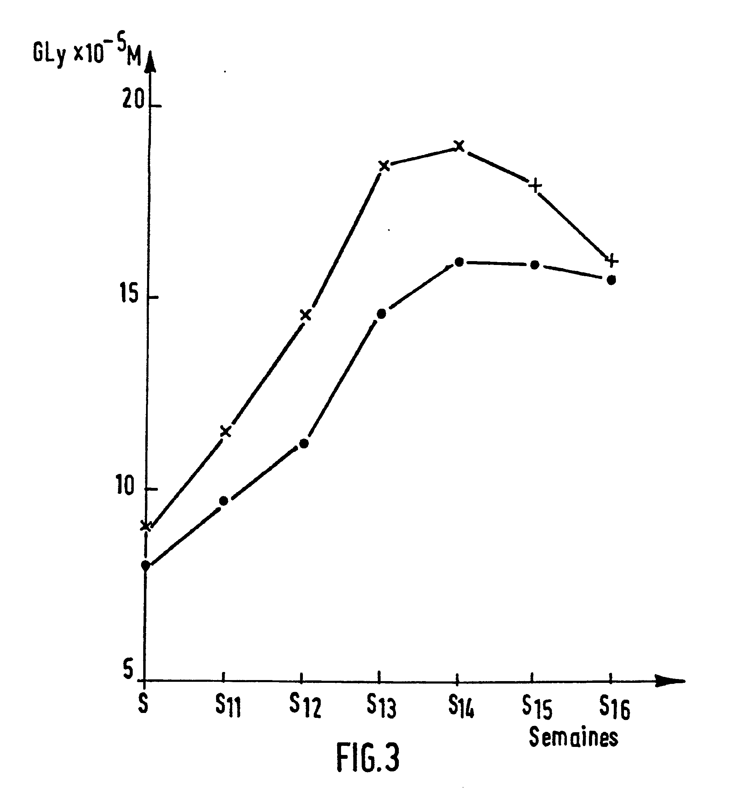

On a représenté sur la figure 3, les courbes de mesure du taux de $NH_2$ avec un brie de lait pasteurisé (courbe x      x) et un brie de lait cru (courbe .      .) par semaine par rapport au taux de $NH_2$ du glycocolle exprimé en méq. x $10^{-5}$M. (De manière classique, les résultats obtenus avec chaque solution de brie sont appréciés en fonction d'une courbe étalon établie avec le glycocolle en opérant dans les mêmes conditions).

D'une manière avantageuse, une telle détermination du taux de groupe $-NH_2$ peut donc être effectuée en utilisant le réactif de l'invention pour transpariser un milieu biologique opalescent.

## EXEMPLE 5 :

Estimation de la vitesse de croissance de bactéries.

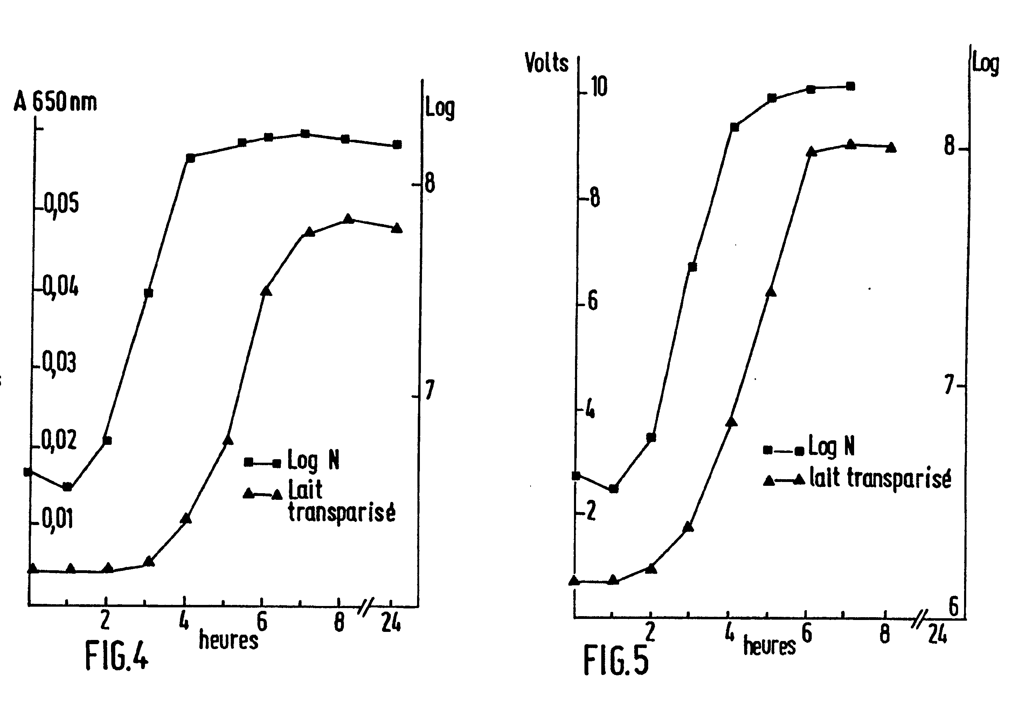

On rapporte sur les figures 4 et 5 les résultats obtenus avec Streptococcus thermophilus CNRZ 302 en opérant selon Kouomègne et al (4).

La figure 4 correspond à la mesure de la densité optique à 650nm en fonction du temps en heures et la figure 5 à la mesure de la tension enregistrée au néphélémètre laser en fonction du temps en heures.

Sur ces figures, les courbes

corespondent au logarithme du nombre de bactéries dénombrées en fonction du temps et les courbes

aux mesures effectuées avec du lait transparisé conformément à l'invention.

Les résultats obtenus sur ces courbes montrent qu'il est possible d'évaluer rapidement le nombre de germes présents dans le lait.

## REFERENCES BIBLIOGRAPHIQUES

(1) Bosset J.O. Blanc, B.H. et Plattner E. Trav. Chim. Aliment. Hyg. (1977), 68 225-239.
(2) Bosset, J.O. Blanc B.H. et Plattner E., Trav. Chim. Aliment. Hyg. (1977), 68, 504-512.
(3) Linden G, Humbert G, Desnouveaux R. et Picard J. Le Lait (1982), 62, 209-219.
(4) Kouomègne R., Bracquart P ; et Linden G, Le Lait (1984), 64, 418-435.

## Revendications

1. Réactif à base d'un mélange de solvant, pour la transparisation d'un milieu biologique, caractérisé en ce qu'il renferme en combinaison :
– une cétone aliphatique constituant un solvant lipophile, soluble en milieu aqueux, stable et permettant une mesure dans le proche UV,
– un agent détergent tensio-actif jouant le rôle de liant, à base d'un dérivé de polyoxyalcoylène non ionique ou d'un sel alcalin amphotère,
– une base et/ou un agent tensio-actif ayant un effet dénaturant vis-à-vis des structures conformationnelles des constituants du milieu biologique à analyser.

2. Réactif selon la revendication 1, caractérisé en ce que la cétone aliphatique est choisie parmi les cétones présentant une constante diélectrique $\Sigma$ de l'ordre de 18,5.

3. Réactif selon la revendication 2, caractérisé en ce que la cétone est une cétone à chaîne linéaire, renfermant au plus 4 atomes de carbones de préférence le butanone-2.

4. Réactif selon l'une quelconque des revendications 1 à 3, caractérisé en ce que l'agent détergent tensio-actif est un dérivé de polyoxyalcoylène non ionique du type éther, alcool ou ester ou un sel alcalin amphotère.

5. Réactif selon la revendication 4, caractérisé en ce que l'agent détergent tensio-actif est constitué par le polyoxyéthylène p,t-octylphénol tel que celui commercialisé sous la marque Triton X.

6. Réactif selon la revendication 4, caractérisé en ce que l'agent détergent tensio-actif est choisi parmi le polyoxyéthylène nonylphénol tel que celui commercialisé sous la marque Triton N, le polyoxyéthylène alcool tel que celui commercialisé sous la marque Brij, le polyoxyéthylène ester de sorbitol tel que celui commercialisé sous la marque Tween, le polyoxyéthylène ester d'acide gras (monolaurate) tel que celui commercialisé sous la marque Span.

7. Réactif selon la revendication 4, caractérisé en ce que l'agent détergent tensio-actif est un sel alcalin amphotère choisi parmi le désoxycholate de sodium et le diiodosalicylate de lithium.

8. Réactif selon l'une quelconque des revendications 1 à 7, caractérisé en ce que la base est de la soude.

9. Réactif selon l'une quelconque des revendications 1 à 8, caractérisé en ce que l'agent tensio-actif est un sel de sodium, de préférence le dodécyl sulfate de sodium.

10. Réactif pour la transparisation d'un milieu biologique, caractérisé en ce qu'il comprend de la butanone-2, du Triton X, de la soude et/ou du dodécylsulfate de sodium.

11. Réactif selon l'une quelconque des revendications précédentes, caractérisé en ce que l'un des groupes, à savoir solvant lipophile et agent détergent tensio-actif d'une part, base et/ou agent dénaturant d'autre part est présent à raison d'au moins 15%, et d'au plus 85%.

12. Réactif selon la revendication 11, caractérisé en ce que le solvant lipophile est présent raison de 15 à 85% en volume, l'agent détergent tensio-actif de 85 à 15% en volume, la base de 0 à 0,5N et l'agent dénaturant de 15 à 0% en volume.

13. Application du réactif selon l'une quelconque des revendications 1 à 12 à la transparisation, aux fins d'analyse, de produits renfermant des protéines et des lipides, plus spécialement des produits alimentaires tels que les produits laitiers, les laits entiers, écrémés, demi-écrémés, en poudre, le lactosérum, les fromages, les levains, les produits carnés, les produits en émulsion du type eau dans l'huile, ou encore des produits cosmétiques notamment les crèmes.

14. Application selon la revendication 13 pour le dosage de groupes spécifiques tels que des fonctions amines, des groupements sulfhydriles et des ponts disulfures ou des éléments minéraux, pour la détermination des activités enzymatiques par exemple, dans le lait et les produits laitiers, de la phosphatase alcaline, de la peroxydase, des protéinases et de la β-D-galactosidase, pour le dénombrement des micro-organismes présents dans un milieu avec identification de ces micro-organismes et l'étude des courbes de croissance de germes.

## Ansprüche

1. Reagens auf der Grundlage eines Lösungsmittelgemisches, zum Transparentmachen eines biologischen Mediums, **dadurch gekennzeichnet**, daß es in Kombination enthält :
– ein aliphatisches Keton als in wäßrigem Medium lösliches lipophiles Lösungsmittel, das stabil ist und eine Messung im nahen UV zuläßt,
– ein grenzflächenaktives, die Rolle eines Vermittlers ausübendes Detergens auf der Grundlage eines nicht-ionischen Derivats von Polyoxyalkoylen oder eines amphoteren Alkalisalzes,
– eine Base und/oder ein grenzflächenaktives Mittel, das gegenüber konformativen Strukturen von Bestandteilen des zu analysierenden biologischen Mediums eine denaturierende Wirkung hat.

2. Reagens gemäß Anspruch 1, **dadurch gekennzeichnet**, daß das aliphatische Keton aus den Ketonen mit einer Dielektrizitätskonstante Σ von der Größenordnung 18,5 ausgewählt ist.

3. Reagens gemäß Anspruch 2, **dadurch gekennzeichnet**, daß das Keton ein geradkettiges Keton mit mindestens 4 Kohlenstoffatomen, vorzugsweise Butanon-2, ist.

4. Reagens gemäß einem der Ansprüche 1 bis 3, **dadurch gekennzeichnet**, daß das grenzflächenaktive Detergens ein nicht-ionisches Derivat des Polyoxyalkoylens vom Ether-, Alkohol- oder Estertyp oder ein amphoteres Alkalisalz ist.

5. Reagens gemäß Anspruch 4, **dadurch gekennzeichnet**, daß das grenzflächenaktive Detergens aus dem Polyoxyethylen-p-(t-octyl)phenol besteht, das unter der Handelsmarke Triton X vertrieben wird.

6. Reagens gemäß Anspruch 4, **dadurch gekennzeichnet**, daß das grenzflächenaktive Detergens aus Polyoxyethylen-Nonylphenol, das unter der Handelsmarke Triton N vertrieben wird, Polyoxyethylen-Alkohol, der unter der Handelsmarke Brij vertrieben wird, Polyoxyethylen-Sorbitolester, der unter der Handelsmarke Tween vertrieben wird, und Polyoxyethylen-Fettsäureester (Monolaurat), der unter der Handelsmarke Span vertrieben wird, ausgewählt ist.

7. Reagens gemäß Anspruch 4, **dadurch gekennzeichnet**, daß das grenzflächenaktive Detergens ein aus Natrium-Desoxycholat und Lithium-Dijodsalicylat ausgewähltes amphoteres Alkalisalz ist.

8. Reagens gemäß einem der Ansprüche 1 bis 7, **dadurch gekennzeichnet**, daß die Base aus Soda besteht.

9. Reagens gemäß einem der Ansprüche 1 bis 8, **dadurch gekennzeichnet**, daß das grenzflächenaktive Mittel ein Natriumsalz, vorzugsweise Natrium-Dodecylsulfat ist.

10. Reagens zum Transparentmachen eines biologischen Mediums, **dadurch gekennzeichnet**, daß es Butanon-2, Triton X, Soda und/oder Natrium-Dodecylsulfat enthält.

11. Reagens gemäß einem der vorstehenden Ansprüche, **dadurch gekennzeichnet**, daß eine der Gruppen, nämlich das lipophile Lösungsmittel und das grenzflächenaktive Mittel einerseits sowie die Base und/oder das denaturierende Mittel andererseits in einer Menge von mindestens 15 und höchstens 85% anwesend sind.

12. Reagens gemäß Anspruch 11, **dadurch gekennzeichnet**, daß die Menge des lipophilen Lösungsmittels 15 bis 85 Volumen-%, die Menge des grenzflächenaktiven Detergens 85 bis 15 Volumen-%, die Stärke der Base 0 bis 0,5N und die Menge des Denaturierungsmittels 15 bis 0 Volumen-% betragen.

13. Verwendung des Reagens nach einem der Ansprüche 1 bis 12 zum Transparentmachen von Produkten zum Zweck der Analyse, die Proteine und Lipide enthalten, insbesondere von Lebensmittelprodukten, wie Milchprodukten, Vollmilch, Magermilch, halbfetter Milch, Milchpulver, Molke, Käse, Hefen, Fleischprodukten, Emulsionen vom Typ Wasser-in-Öl oder auch von Kosmetikprodukten wie Cremes.

14. Verwendung gemäß Anspruch 13 zur Bestimmung spezifischer Gruppen wie Aminfunktionen, Sulfhydrilgruppen und Disulfidbrücken oder Mineralelementen, zur Bestimmung von Enzymaktivitäten zum Beispiel in Milch und Milchprodukten, von alkalischer Phosphatase, von Peroxydase, von Proteinasen und von β-D-Galactosidase, zur Bestimmung der Anzahl an Mikroorganismen in einem Medium sowie zu deren Identifizierung und zum Zweck des Studiums von Wachstumskurven von Keimen.

## Claims

1. Reagent based on a mixture of solvents to render transparent a biological medium, characterized in that it contains in combination :
– an aliphatic ketone constituting a lipophilic solvent, soluble in aqueous media, stable and making possible measurements in the near UV,
– a detergent playing the role of binding agent and consisting of a non-ionic polyoxyalkylene derivative or an amphoteric alkaline salt,
– a base and/or a surface active agent possessing the property of being able to denature the conformational structures of the constituents of the biological medium to be analyzed.

2. Reagent according to claim 1, characterized in that the aliphatic ketone is chosen from among ketones possessing a dielectric constant, $\varepsilon$, of the order of 18.5.

3. Reagent according to claim 2, wherein said ketone is a ketone with a linear chain containing up to 4 carbon atoms, preferably butanone-2.

4. Reagent according to anyone of claims 1 to 3, wherein said surface active detergent is a non-ionic polyoxyalkylene derivative of the ether, alcohol or ester type or an amphoteric alkaline salt.

5. Reagent according to claim 4, wherein said surface active detergent is constituted by a polyethylene glycol 4-tert-octylphenyl ether such as the one sold under the trademark Triton X.

6. Reagent according to claim 4, wherein said surface active detergent is chosen from among nonyl phenoxypolyethoxyethanol derivatives, polyethylene glycol ether derivatives, polyoxyethylene sorbitol ester derivatives and polyoxyethylene esters of fatty acids.

7. Reagent according to claim 4, wherein said surface active detergent is an amphoteric alkaline salt chosen from among sodium desoxycholate and lithium diiodosalicylate.

8. Reagent according to anyone of claims 1 to 7, wherein said base is sodium hydroxyde.

9. Reagent according to anyone of claims 1 to 8, wherein said surface active agent is a sodium salt, preferably sodium dodecyl sulfate.

10. Reagent for making a biological medium transparent, characterized in that it contains butanone-2, Triton X, sodium hydroxyde and/or sodium dodecyl sulfate.

11. Reagent according to anyone of the preceding claims, wherein one of the groups, constituted by the lipophilic solvent and the surface active detergent on the one hand, the base and/or the denaturating agent on the other, is present in an amount not less that 15% and not more than 85%.

12. Reagent according to claim 11, wherein the lipophilic solvent is present to the extent of from 15% to 85% by volume, the detergent from 85% to 15% by volume, the base from 0 to 0.5 N and the denaturating agent from 15% to 0% by volume.

13. Use of the reagent according to anyone of claims 1 to 12, for making transparent, in view of their analysis, particularly foodstuffs such as milk products, whole milks, skimmed milks, semi-skimmed milks, powered milks, whey, cheeses, leavens, meat products, emulsions of the oil-in-water type, or cosmetic products, particularly creams.

14. Use according to claim 13 for the dosage of specific groups such as amino functions, sulfhydril groups and disulfide bridges or mineral elements for the determination of the enzymatic activities for example in milk and milk products, of alkaline phosphatase, peroxydase, proteinase and β-galactosidase, for the counting of the micro-organisms present in a biological medium with the identification of these micro organisms and the study of their growth curves.

FIG.1a

FIG.1b

FIG.1c

FIG.2

FIG.3

FIG.4

FIG.5

EP 0 246 978 B1